# FASCICULE DE BREVET EUROPEEN

(11) **EP 2 773 332 B1**
(45) Date de publication et mention de la délivrance du brevet: **29.11.2017**
(21) Numéro de dépôt: 12798863.2
(22) Date de dépôt: 31.10.2012
(51) Int. Cl.: A61K 9/51, A61K 9/127, A61K 49/18

(54) **ASSOCIATION D'UNE NANOPARTICULE COMPORTANT UN COEUR CHARGE EN PHOSPHOLIPIDE AVEC UNE PROTÉINE - UTILISATION POUR LA DÉLIVRANCE IN VITRO DE LADITE PROTÉINE**
KOMBINATION AUS EINEM NANOPARTIKEL MIT EINEM GELADENEN KERN AUS PHOSPHOLIPID MIT EINEM PROTEIN UND VERWENDUNG DAVON ZUR IN-VITRO-ABGABE DIESES PROTEINS
COMBINATION OF A NANOPARTICLE COMPRISING A CHARGED CORE MADE OF PHOSPHOLIPID WITH A PROTEIN - USE FOR THE IN VITRO DELIVERY OF SAID PROTEIN

(30) Priorité: 03.11.2011 FR 1103333
(43) Date de publication de la demande: 10.09.2014
(73) Titulaire: Université de Droit et de la Santé de Lille 2, 59800 Lille (FR); Centre Hospitalier Regional Universitaire de Lille, 59037 Lille Cédex (FR)
(72) Inventeur: BETBEDER, Didier, F-59263 Houplin Ancoisne (FR); LEFEBVRE, Bruno, F-59700 Marcq-en-baroeul (FR)
(74) Mandataire: IP Trust
(86) Numéro de dépôt international: PCT/IB2012/056064
(87) Numéro de publication internationale: WO 2013/065002

(56) Documents cités:
- WO-A1-2005/099889
- WO-A2-2007/133616
- WO-A2-2009/042895
- WO-A2-2010/078569
- US-A1- 2004 022 840
- US-A1- 2010 331 819
- JALLOULI ET AL: "Influence of surface charge and inner composition of porous nanoparticles to cross blood-brain barrier in vitro", INTERNATIONAL JOURNAL OF PHARMACEUTICS, ELSEVIER BV, NL, vol. 344, no. 1-2, 27 septembre 2007 (2007-09-27), pages 103-109, XP022275358, ISSN: 0378-5173, DOI: 10.1016/J.IJPHARM.2007.06.023
- SANTOS N C ET AL: "Structural characterization of organized systems of polysaccharides and phospholipids by light scattering spectroscopy and electron microscopy", CARBOHYDRATE RESEARCH, PERGAMON, GB, vol. 300, no. 1, 9 mai 1997 (1997-05-09), pages 31-40, XP004064935, ISSN: 0008-6215, DOI: 10.1016/S0008-6215(97)00025-6
- GOMES J F P S ET AL: "Lipid/particle assemblies based on maltodextringum arabic core as bio-carriers", COLLOIDS AND SURFACES. B, BIOINTERFACES, ELSEVIER, AMSTERDAM, NL, vol. 76, no. 2, 1 avril 2010 (2010-04-01), pages 449-455, XP026888221, ISSN: 0927-7765, DOI: 10.1016/J.COLSURFB.2009.12.004 [extrait le 2010-01-13]

## Description

### DOMAINE TECHNIQUE

La présente invention concerne une association entre une nanoparticule comprenant ou consistant en (a) un coeur solide et poreux, formé d'un polymère obtenu par réaction entre une maltodextrine et un composé époxyde, des fonctions ammonium quaternaire étant greffées sur ledit polymère pour lui conférer une charge positive, et (b) un lipide de charge ionique négative, et une protéine ou un fragment de protéine qui est fixé, notamment adsorbé(e) sur ledit coeur, pour la délivrance notamment *in vitro* de ladite protéine dans une cellule vivante.

### ART ANTERIEUR

Il existe un besoin d'outils d'étude des mécanismes de fonctionnement cellulaires. Une meilleure compréhension de ces mécanismes permettrait en effet d'aboutir à de nouvelles thérapies qui prendraient en charge la cellule elle-même.

Massignani et al. (Nature Precedings, 8 May 2010) décrit un procédé de délivrance d'un anticorps dans une cellule, basé sur l'utilisation de nano capsules de copolymères sensibles au pH. Ces nano capsules sont encore appelées *« polymersome* ». Des copolymères particuliers sont capables de former, dans un milieu dont le pH est sensiblement égal au pH physiologique, des nano capsules. Des anticorps peuvent être encapsulés dans ces nano capsules pour être délivrés dans des cellules. Des nano capsules remplies d'anticorps sont introduites dans des cellules dans lesquelles elles sont prises en charge par les mécanismes de l'endocytose. Lors de l'endocytose, les nano capsules sont soumises à une brusque chute de pH. Cette chute de pH provoque la dislocation des nano capsules, les copolymères formant à ce pH des chaînes polymériques. Cette transformation des copolymères augmente la pression osmotique ce qui aboutit à la rupture de la membrane des endosomes provoquant la libération des anticorps dans le cytoplasme de la cellule. Cependant, ce procédé, qui nécessite la formation des nano capsules puis l'encapsulation des anticorps, est assez complexe. En effet le procédé nécessite la préparation et caractérisation d'un copolymère et l'ajustement du pH à 6 avant d'ajouter l'anticorps à délivrer. Le mélange copolymère anticorps doit être soniqué pendant 10 minutes. Les nano capsules contenant les anticorps sont ensuite extraites et séparées des autres composants par chromatographie d'exclusion sur Sepharose 4B. De plus, la concentration en nano capsules et le taux d'encapsulation de l'anticorps doivent être déterminés avant utilisation de la solution contenant les nano capsules préparées. JALLOULI ET AL (INTERNATIONAL JOURNAL OF PHARMACEUTICS, ELSEVIER BV, NL, (2007-06-22)) décrit l'évaluation de la capacité de nanoparticules poreuses à traverser la barrière hémato-encéphalique. Ce document décrit des nanoparticules (NPs) comprenant un coeur (core) formé d'un polymère obtenu par réaction entre une maltodextrine et de l'épichlorhydrine, dc l'hydroxycholine étant greffée sur le polymère et du DPPG étant contenu dans le coeur. Ce document suggère également que les nanoparticules précitées pourraient être utilisées pour la délivrance de médicaments (" NPs as potential drug carriers ").

SANTOS N. C. ET AL (CARBOHYDRATE RESEARCH, PERGAMON, GB, (1997-05-09)), décrit la caractérisation structurale de nanoparticules du type biovecteurs formées d'un coeur polysaccharidique auquel des phospholipides peuvent être ajoutés. Le coeur de ces biovecteurs peut avoir une charge positive, neutre ou négative. Ce document décrit notamment des biovecteurs qui comprennent un coeur formé d'un polymère obtenu par réaction entre une maltodextrine et de l'épichlorhydrine et où de l'hydroxycholine est greffée sur le polymère pour lui conférer une charge positive. Il existe donc un besoin en moyens simples et efficaces, permettant la délivrance de protéines, en particulier des anticorps, dans des cellules, qui puissent être utilisés dans des procédés.

### DESCRIPTION DE L'INVENTION

Un but de la présente invention est de proposer un moyen à base de nanoparticules telles que décrites dans l'invention, permettant d'introduire, de délivrer ou de transférer un anticorps ou un fragment fonctionnel d'anticorps dans le cytosol d'une cellule vivante de manière à pouvoir observer les interactions entre cet anticorps ou un fragment fonctionnel d'anticorps et la cellule, la protéine ou le fragment introduit dans la cellule modifiant le fonctionnement de la cellule ou pouvant marquer certains compartiments de la cellule pour des applications en imagerie cellulaire.

Ce but est atteint au moyen d'une association entre une nanoparticule et un anticorps ou un fragment fonctionnel d'anticorps, ladite nanoparticule comprenant (a) un coeur solide et poreux, formé d'un polymère obtenu par réaction entre une maltodextrine et un composé époxyde, des fonctions ammonium quaternaire étant greffées sur ledit polymère pour lui conférer une charge positive et (b) un phospholipide de charge ionique négative contenu dans ledit coeur. Selon l'invention, de manière caractéristique, ledit anticorps ou ledit fragment fonctionnel d'anticorps est fixé sur ledit coeur. Par fixé, on entend, que l'anticorps ou le fragment fonctionnel d'anticorps est lié à la surface du coeur de la nanoparticule (sans limitation du type de force et interaction qui permettent de lier le coeur à ladite protéine ou du fragment de protéine). L'anticorps ou le fragment fonctionnel d'anticorps peut, par exemple, être adsorbé sur ledit coeur. Le terme « adsorbé » peut désigner notamment, une liaison non covalente, par exemple une liaison de faible énergie, de type van der Waals (hydrophobe) ou une liaison ionique.

Avantageusement, l'anticorps est fixé à la surface du coeur de la nanoparticule par des interactions de faible énergie (polaire, apolaire ou ionique).

Les inventeurs ont en effet découvert que les protéines de taille supérieure à la section des pores du coeur ne peuvent pénétrer dans ce dernier. Les protéines étant globalement de charge négative, lorsqu'elles ne peuvent pas pénétrer dans le coeur, elles s'associent à la surface de ce dernier, qui est chargé positivement dans le cas de la présente invention. Les inventeurs ne sont néanmoins pas liés à cette explication. Ainsi, les inventeurs ont découvert que notamment les protéines de masse moléculaire supérieure ou égale à 100KDa ne pouvaient pas pénétrer dans le coeur des nanoparticules comportant un coeur tel que précité, du fait, probablement, de leur taille trop importante. Des protéines de tailles plus petite que précitée mais étant agrégées ou agglomérées, peuvent également rester liées à la surface du coeur de la particule du fait de la taille trop importante de l'agrégat ou de l'agglomérat. Le terme « protéine » ou « fragment de protéine » englobe donc à la fois des protéines et des fragments en tant que tels mais aussi des entités qui sont des agglomérats de protéines ou de peptides.

Concernant la taille des protéines ou fragments de protéines, l'invention ne se limite pas à une taille précise. La position de la protéine ou du fragment de protéine par rapport à la nanoparticule dépend de la taille de la protéine ou du fragment par rapport à la taille des pores du coeur de la nanoparticule. Il est donc à la portée de l'Homme du Métier de déterminer, en mélangeant la nanoparticule telle que précitée avec une protéine ou un fragment de protéine (éventuellement dans un solvant), si la protéine ou le fragment de protéine présente une taille supérieure à la section des pores du coeur. Une méthode utilisable est explicitée ci-après et met en oeuvre l'augmentation de taille de la nanoparticule ainsi que la modification de sa charge.

Le type de coeur précité s'est révélé particulièrement performant pour la délivrance d'une protéine. En effet, la cytotoxicité de la nanoparticule comportant un tel coeur peut être contrôlée en fonction de la concentration en nanoparticules mises en contact avec la cellule, de la densité de charge de la nanoparticule, des conditions de cultures des cellules, et de la durée de contact entre les nanoparticules et les cellules. Par ailleurs, de telles nanoparticules se sont révélées être très stables dans le temps à température ambiante, et aptes à être stérilisées sans que leurs propriétés, en particulier quand à la délivrance de la protéine, ne soient altérées.

Le document FR 2 803 517 décrit la fabrication de nanoparticules cationiques comportant un coeur à base de maltodextrine greffée telles que précité. Le phospholipide est contenu dans le coeur. De telles nanoparticules sont utilisées pour la préparation d'un complexe comportant un principe actif destiné à être administré par voie mucosale. Le principe actif, qui peut être une protéine, est toujours incorporé dans le coeur, soit lors de la formation du polymère constitutif de ce dernier, soit lors de l'introduction du phospholipide dans les porosités du coeur. Par ailleurs, l'anticorps n'est pas cité parmi les principes actifs associables à la nanoparticule.

La publication de Paillard et al. [2010 ; Pharm Res, Issue 1 (2010), 126-133)]. décrit des nanoparticules comportant un coeur identique à celui des particules de l'invention qui est également chargé en phospholipide. Ces nanoparticules comportent dans leur coeur, en plus du phospholipide, une protéine (une albumine). La publication démontre que la protéine n'est pas adsorbée à la surface du coeur mais incorporée dans ce dernier. Par ailleurs, ces nanoparticules n'ont jamais été mises au contact de cellules. Or, le comportement des nanoparticules par rapport à une cellule vivante est très dépendant de la nature du coeur et du lipide contenu dans ce dernier.

La publication « Evaluation of effect of charge and lipid coating on ability of 60-nm nanoparticules to cross an in vitro model of the blood-brain barrier » publiée dans la revue The Journal of Pharmacology and Experimental Therapeutics décrit des nanoparticules comportant un coeur identique à celui des nanoparticules de l'invention. Ces particules ne comportent pas de phospholipide incorporé dans le coeur ; le coeur est recouvert d'un mélange de lécithines (diacyl phosphatidyl choline de charge globale neutre (zwitterioniques). Ces particules sont capables de pénétrer dans une cellule et de la traverser, par transcytose, sans délivrance dans le cytosol. La cellule les prend en charge et les libère ensuite comme tout corps étranger. Ces nanoparticules sont capables ainsi de franchir *in vitro* la barrière hémato encéphalique (BHE) dans des conditions sans sérum mais, en présence de globules rouges, il a été montré qu'elles ne pouvaient plus pénétrer dans les cellules de la BHE (voir publication précitée).

La protéine liée à la surface du coeur de la nanoparticule de l'invention peut être choisie parmi les anticorps, les fragments fonctionnels d'anticorps. Avantageusement, la protéine adsorbée est un anticorps, plus particulièrement un anticorps du type IgG et plus particulièrement PDX-1, qui peut éventuellement également être nommé anti-PDX-1.

Les anticorps ont une demi-durée de vie longue dans l'organisme. Normalement, les anticorps ne pénètrent pas dans les cellules mais agissent au niveau de la paroi de ces dernières. La présente invention a pour avantage de permettre l'introduction d'un anticorps (ou fragment fonctionnel d'anticorps) dans une cellule, cet anticorps conservant de plus, et de manière surprenante, ses fonctionnalités au sein de la cellule, en termes de reconnaissance et/ou de fixation de l'antigène cible. De plus, les inventeurs ont en effet montré que l'association selon l'invention permet la délivrance de la protéine (un anticorps, en particulier) dans le cytosol d'une cellule, la protéine interagissant alors avec la cellule, et modifiant le fonctionnement biologique de cette dernière.

Avantageusement, le phospholipide de charge ionique négative, est choisi parmi le 1, 2-diacylyl-sn-glycéro-3-phosphatidylglycérol, le 1, 2-diacylyl-sn-glycero-3-phosphatidylsérine, le 1, 2-dipalmitoyl-sn-glycéro-3-phosphatidylglycérol et le 1, 2-diacylyl-sn-glycéro-3-phosphatidylsérine.

Avantageusement, la nanoparticule a un taux de charge lipidique supérieur ou égal à environ de 10 à 100%. Dans un mode de réalisation particulier, le taux de charge lipidique des nanoparticules de l'invention est compris entre 50 et 80%, de préférence entre 65 et 70%. Le taux de charge lipidique est défini comme étant le rapport massique : masse de lipide incorporé/masse de la nanoparticule, ce taux étant une valeur moyenne établie sur une masse donnée de nanoparticules et une masse donnée de lipide.

La taille des nanoparticules utilisables dans le cadre de l'invention n'est pas limitée. Toutefois, dans un mode de réalisation particulier, les nanoparticules présentent une taille moyenne comprise entre 5 nm et 10 microns (µm), de préférence entre 5 nm et 1 µm, entre 5 et 100 nm, entre 10 et 100 nm, entre 20 et 100 nm ou entre 40 et 60nm. Cette taille peut être déterminée par tout moyen connu, en particulier par diffusion dynamique de la lumière à l'aide du NanoZS (Malvern). La méthode précitée permet d'obtenir la taille moyenne des nanoparticules qui est calculée à partir des dimensions de ces dernières mesurées selon les trois directions orthogonales. Cette taille est une valeur moyenne calculée sur un ensemble (ou population) de nanoparticules à partir des trois dimensions des nanoparticules et qui dépend notamment de la forme de la nanoparticule.

La forme des nanoparticules utilisables dans le cadre de l'invention n'est pas limitée. Il est connu que la forme de la nanoparticule peut être déduite de l'indice de polydispersité. En effet, la taille moyenne étant calculée selon trois directions orthogonales, elle prend en compte la forme des nanoparticules. Ainsi, plus l'indice de polydispersité est grand, plus les nanoparticules ont des formes ou des tailles variables ou sont, par exemple, sous la forme de bâtonnets dont les trois dimensions sont très différentes l'une de l'autre. A l'inverse, plus l'indice de polydispersité est bas, plus l'ensemble de nanoparticules est homogène en taille. Dans un mode de mise en oeuvre particulier, l'ensemble de nanoparticules utilisable dans le cadre de l'invention présente un indice de polydispersité inférieur ou égal à 0,3. En effet, un indice de polydispersité bas (inférieur ou égal à 0,3) indique que les trois dimensions des nanoparticules sont sensiblement égales et que les nanoparticules sont donc statistiquement sphériques. Ainsi, les inventeurs ont mis en évidence qu'un ensemble de nanoparticules sensiblement sphériques (indice de polydispersité inférieur ou égal à 0,3) influence la délivrance et permet une libération plus facile de la protéine ou du polypeptide dans le cytosol de la cellule. D'autres formes de nanoparticules permettent néanmoins d'aboutir également à la libération de la protéine et/ou du polypeptide dans la cellule.

La taille, la forme et l'indice de polydispersité décrits dans la présente invention peuvent être déterminés et calculés par tout moyen connu, en particulier par les méthodes décrites dans la publication de Paillard et al. [2010 ; Pharm Res, Issue 1 (2010), 126-133)].

Avantageusement, pour une utilisation en imagerie, un élément choisi parmi le coeur de ladite nanoparticule, le phospholipide contenu dans ledit coeur et ladite protéine ou ledit fragment de protéine est associé à un produit de marquage. Ce produit de marquage peut être, par exemple, un radio-marqueur ou un marqueur fluorescent.

La présente invention concerne également l'utilisation d'une nanoparticule selon l'invention pour la délivrance de ladite protéine ou dudit fragment de protéine fixé(e) sur ledit coeur dans le cytosol d'une cellule, ladite protéine ou ledit fragment de protéine ainsi libéré dans le cytosol interagissant avec le fonctionnement de ladite cellule, ou pouvant marquer ses composants (lorsqu'un produit de marquage est utilisé).

La présente invention concerne, en outre, un procédé de délivrance *in vitro* d'un anticorps ou d'un fragment fonctionnel d'anticorps dans le cytosol d'une cellule, selon lequel
- on mélange, éventuellement dans un solvant adapté, un anticorps ou un fragment fonctionnel d'anticorps avec une nanoparticule qui comprend (a) un coeur solide et poreux, formé d'un polymère obtenu par réaction entre une maltodextrine et un composé époxyde, des fonctions ammonium quaternaire étant greffées sur ledit polymère pour lui conférer sa charge positive et (b) un phospholipide de charge ionique négative contenu dans ledit coeur, et
- on met en contact ledit mélange avec une cellule vivante.

De façon surprenante, les inventeurs ont mis en évidence que le mélange des nanoparticules telles que décrites dans la présente demande avec une protéine ou un fragment de protéine, éventuellement au sein d'un solvant, permettait d'obtenir des nanoparticules associées à ladite protéine qui sont capables de délivrer ladite protéine ou ledit fragment de protéine dans le cytosol d'une cellule vivante, par simple contact avec cette cellule. De façon intéressante, la protéine associée à la nanoparticule est libéré(e) dans le cytosol et interagit avec la cellule, modifiant le fonctionnement de cette dernière. La durée de contact pour obtenir la libération de la protéine dépend du type de protéine, du type de cellule et des conditions de culture.

Selon le procédé de l'invention, le terme « associé(e) » signifie que l'anticorps ou le fragment fonctionnel d'anticorps peut être fixé, lié à la surface de la nanoparticule. La nanoparticule peut donc également comporter des anticorps liés (fixés, éventuellement par adsorption) à la surface de ce dernier.

La protéine est choisie, par exemple, parmi les anticorps, les fragments d'anticorps, les enzymes, les facteurs de transcription, les facteurs de régulation, les cytokines, les protéines de transport, les protéines nucléaires, les protéines mitochondriales et les protéines biologiquement actives.

Dans un mode de mise en oeuvre particulier, la protéine est un anticorps, plus particulièrement un anticorps de type IgG et, plus particulièrement, l'anticorps PDX-1, qui peut éventuellement également être nommé anti-PDX-1.

Avantageusement, le phospholipide est choisi parmi les phospholipides anioniques tels que le 1, 2-diacylyl-sn-glycéro-3-phosphatidylglycérol, le 1, 2-diacylyl-sn-glycero-3-phosphatidylsérine, le 1, 2-dipalmitoyl-sn-glycéro-3-phosphatidylglycérol et le 1, 2-diacylyl-sn-glycéro-3-phosphatidylsérine.

Par solvant adapté, on entend, au sens de la présente invention, tout solvant utilisable *in vitro* et/ou *in vivo* sans dommage pour la cellule ou l'organisme. En particulier, un solvant pharmaceutiquement acceptable peut être utilisé.

Dans un mode de réalisation particulier, le solvant utilisé pour la formation du mélange est un solvant protique, polaire par exemple l'eau ou apolaire.

La présente demande concerne également l'association entre une nanoparticule comprenant (a) un coeur solide et poreux, formé d'un polymère obtenu par réaction entre une maltodextrine et un composé époxyde, des fonctions ammonium quaternaire étant greffées sur ledit polymère pour lui conférer une charge positive,(b) un phospholipide de charge ionique négative contenu dans ledit coeur et (c) un anticorps ou un fragment d'anticorps associé à ladite nanoparticule.

Le terme « associé » signifie, notamment que l'anticorps ou le fragment d'anticorps est soit contenu dans le coeur, soit lié à la surface du coeur (indépendamment du type de force qui créé la liaison), soit à la fois contenu dans le coeur et lié (fixé) à la surface de ce dernier.

Les inventeurs ont en effet montré qu'une telle association nanoparticule/anticorps permettait la délivrance fonctionnelle de l'anticorps ou du fragment d'anticorps dans le cytosol de la cellule.

L'anticorps utilisé en association avec la nanoparticule telle que précitée peut être un anticorps du type IgG et plus particulièrement PDX-1, qui peut éventuellement également être nommé anti-PDX-1.

Le phospholipide contenu dans le coeur de la nanoparticule associée à un anticorps telle que précité, peut être choisi parmi le 1, 2-diacylyl-sn-glycéro-3-phosphatidylglycérol, le 1, 2-diacylyl-sn-glycero-3-phosphatidylsérine, le 1, 2-dipalmitoyl-sn-glycéro-3-phosphatidylglycérol et le 1, 2-diacylyl-sn-glycéro-3-phosphatidylsérine.

La présente invention concerne également une composition pharmaceutique comprenant, en tant que composant actif, une association nanoparticule-anticorps ou fragment fonctionnel d'anticorps, selon l'invention et un solvant pharmaceutiquement acceptable. En effet, la protéine ou le fragment de protéine pouvant pénétrer dans les cellules, l'association précitée peut servir en thérapie, par exemple, pour le traitement de maladies causées par la production insuffisante ou inexistante d'une protéine donnée. La mucoviscidose, la maladie de Duchenne et certains types de cancers peuvent être cités comme exemples de telles pathologies. La composition selon l'invention peut également servir au traitement de maladies causées par des protéines mal repliées. La maladie de Parkinson, la sclérose en plaques et la maladie d'Alzheimer peuvent être citées comme exemples de telles pathologies. L'association objet de l'invention peut, en délivrant de manière fonctionnelle la protéine manquante ou déficiente dans le cytosol de la cellule de rétablir le fonctionnement normal de cette dernière.

Avantageusement, la composition est formulée pour être adaptée à un usage par voie mucosale, en particulier pour les muqueuses des voies aériennes.

La présente demande, ses caractéristiques et les différents avantages qu'elle procure seront mieux compris à la lecture de la description qui suit et des exemples cités à titre non limitatif et qui font référence aux figures annexées sur lesquelles :
- la Figure 1 représente l'expression des gènes Ins1 (A) et Ins2 (B) déterminées 48h après incubation des différentes associations nanoparticule/anticorps testées avec les cellules ; Ct : contrôle négatif.
- la **Figure 2** représente l'expression des gènes Ins1 (A) et Ins2 (B) déterminées 72h après incubation des différentes associations nanoparticule/anticorps testées avec les cellules ; et
- la **Figure 3** représente l'expression des gènes Ins1, Ins2, Pdx1 et Mafa, déterminées 72h après incubation des différentes associations nanoparticule/anticorps testées avec les cellules ;
- les **Figures 4a et 4b** représentent respectivement la variation de la taille et la variation du potentiel zéta des nanoparticules comportant un coeur tel que précité et chargé en phospholipide lors de leur association avec de l'albumine de sérum bovin ;
- les **Figures 5a et 5b** représentent respectivement la variation de la taille et la variation du potentiel zéta des nanoparticules comportant un coeur tel que précité et chargé en phospholipide lors de leur association avec de l'ovalbumine ; et
- les **Figures 6a et 6b** représentent la variation de la taille et la variation du potentiel zéta des nanoparticules comportant un coeur tel que précité et chargé en phospholipide lors de leur association avec un anticorps anti-transferrine.

### EXEMPLES

### Préparation des nanoparticules

Les nanoparticules sont préparées, par exemple, selon le protocole décrit par Paillard et al. [2010 ; Pharm Res, Issue 1 (2010), 126-133)].

### Exemple 1: Préparation de nanoparticules hydrophiles cationiques submicroniques à base de maltodextrine greffée par des fonctions ammonium quaternaire (fabrication du coeur).

50 g d'une maltodextrine (Glucidex 6, hydrolysat de polysaccharide Mw : 3400, Roquette Frères, France) sont solubilisés dans 80 ml d'eau puis 20 ml de soude 10M sont ajoutés. Dans la solution alcaline de polysaccharide maintenue sous agitation, 3,54 g d'épichlorhydrine (38,3 mmol) sont ajoutés. Après une nuit d'agitation douce à température ambiante, la solution gélifie.

31,2 g de glycidyl triméthyl ammonium (200 mmol) sont ajoutés au gel et la réaction est laissée 8 heures sous agitation. Après la fin de la réaction, 1 litre d'eau est ajouté et le pH de la solution est amené entre 5 et 7 à l'aide d'acide acétique. La suspension est pré-homogénéisée à l'aide d'un Ultra-Turrax T-25. La suspension de particules est alors homogénéisée (Minilab H-80) à une pression variant entre 500 et 900 bars pour obtenir des particules ayant une taille inférieure à 100nm.

Les particules sont ensuite purifiées par ultrafiltration sur une membrane de 30 kDa (système Minissette, Filtron, USA). La suspension de particules est ensuite purifiée par micro filtration sur membrane de 0,2 m (Spiral-Cap 0,2 m, Gelman, USA) et stockée à 4 °C.

Les caractéristiques de la suspension sont :
- Taille (Coulter N4D) : 40- 80 nm (S. D= 20 nm)
- Charge (Analyse élémentaire) : 2mmol/g
- Potentiel Z (Malvern série 3000 Tampon phosphate 15 mM pH 7) : + 25 +/- 10 mV
- Concentration : 17 g/l

### Exemple 2 : introduction du lipide dans le coeur précédemment formé

24 ml d'une solution à 17 g/l (408 mg de particules) en matrice hydrophile cationique préparée suivant l'exemple 1 sont maintenus sous agitation à 80 C. A l'aide d'une pompe péristaltique, 10,2 ml d'une solution éthanolique de DPPG (DPPG : 1, 2-dipalmitoyl-sn-glycéro-3-phosphatidylglycérol ou dipalmitoylphosphatidyl glycérol) (commercialisé par la société Lipoid®) 16 g/l (éthanol : eau 88 : 12, DPPG 163 mg) sont ajoutés lentement dans la solution de polysaccharide cationique. Après l'ajout de la totalité du DPPG, la solution est laissée sous agitation à 80 C durant 30 min. La solution est purifiée par ultrafiltration sur une membrane ayant un seuil de filtration de 30 KDa.

Il en résulte une suspension de matrices amphiphiles submicroniques avec un rapport matrice : lipides de 40 % w/w. En tenant compte du taux de charge de la matrice (2 mmol / g) et du poids molaire du DPPG (722 Da), on peut conclure que le taux de neutralisation de la charge cationique par le DPPG est de 26 %. Il en résulte 34 ml d'une suspension de particules submicroniques (40-80 nm mesurées au Coulter N4D) à 16,5 g /l. Le potentiel Z de ces matrices amphiphiles reste largement cationique (+ 15 mV mesuré dans du tampon phosphate 15mM pH 7).

### Délivrance d'un anticorps dans des cellules RINm5F

Les exemples qui suivent ont pour but de tester l'effet de la délivrance de l'anticorps anti PDX-1 sur l'expression du gène de l'insuline. L'introduction de cet anticorps dans la cellule permet d'inhiber spécifiquement l'activité de PDX-1 qui est un facteur de transcription impliqué dans la régulation de l'expression des gènes de l'insuline Ins1 et Ins2 chez les rongeurs. L'exemple est réalisé sur la lignée RINm5F provenant d'un insulinome de rat. Cette lignée est difficilement transfectable avec les techniques couramment utilisées dans les laboratoires. Par exemple, l'utilisation de la lipofectamine 2000 avec du siRNA anti-PDX1 (Santa Cruz, US) n'a pas donné de résultats significatifs.

### Protocole de culture

Les cellules RINm5F à 70% confluence sont rincées au PBS et trypsinées. La suspension cellulaire est rassemblée et centrifugée pendant 5 minutes à une vitesse de 1200 rpm. Le culot est ensuite repris dans un volume de 5mL de milieu RPMI 1640 (ATCC 30-2001) complémenté avec 10% de SVF (sérum de veau foetal) et 1 % de pénicilline/streptomycine.

La suspension cellulaire de cellules RINm5F est répartie dans des puits d'une plaque de culture de 12 puits, à raison de 250 000 cellules/puits. Les cellules sont cultivées pendant 24 h de manière à adhérer au fond des puits.

### Préparation des formulations à tester

Dans des Eppendorfs distincts, sont réparties, sous la hotte, pendant 1 h d'incubation à TA (température ambiante), différentes solutions d'anticorps, de nanoparticules seules et de nanoparticules sur lesquelles sont fixés différents anticorps. Ces solutions sont utilisées pour la mise en oeuvre du procédé de l'invention.
- 5µg de NPL70
- 5µg NP+
- 1µg IgG (IgG anti-lapin non spécifique)
- 2.5µg IgG
- 5µg IgG
- 1µg d'anticorps anti PDX-1 (α PDX-1)
- 2.5µg α PDX-1
- 5µg α PDX-1
- 5µg NP+/1µg IgG
- 5µg NP+/2.5µg IgG
- 5µg NP+/5µg α IgG
- 5µg NPL70/1µg IgG
- 5µg NPL70/2.5µg IgG
- 5µg NPL70/5µg α IgG
- 5µg NP+/1µg α PDX-1
- 5µg NP+/2.5µg α PDX-1
- 5µg NP+/5µg α PDX-1
- 5µg NPL70/1µg α PDX-1
- 5µg NPL70/2.5µg α PDX-1
- 5µg NPL70/5µg α PDX-1

Après 1 heure d'incubation des solutions mentionnées ci-dessus, on ajoute 0,5mL de milieu contenant du SVF pendant 1 heure à TA. Les préparations sont ensuite réparties sur les cellules RINm5F pendant 6 heures d'incubation avec du milieu contenant du SVF. Après ce temps d'incubation les puits sont lavés et du milieu frais est ajouté pendant une durée de 48h ou 72h. Un contrôle négatif (Ct) représente les cellules testées seules, sans incubation avec une solution.

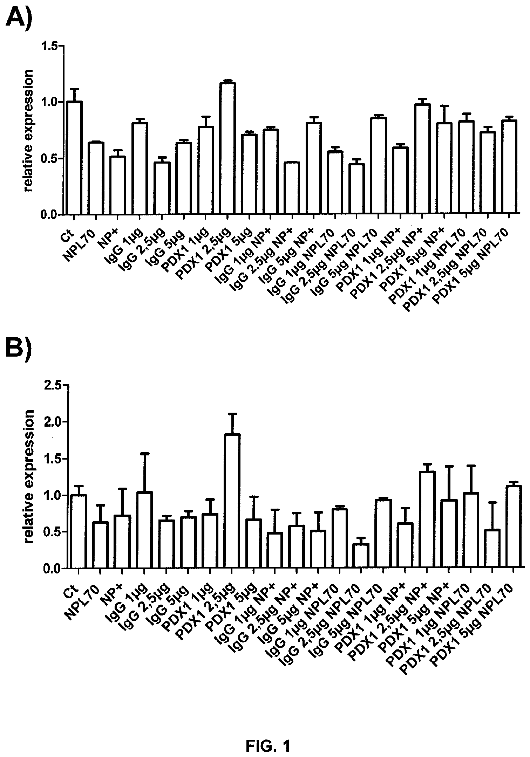

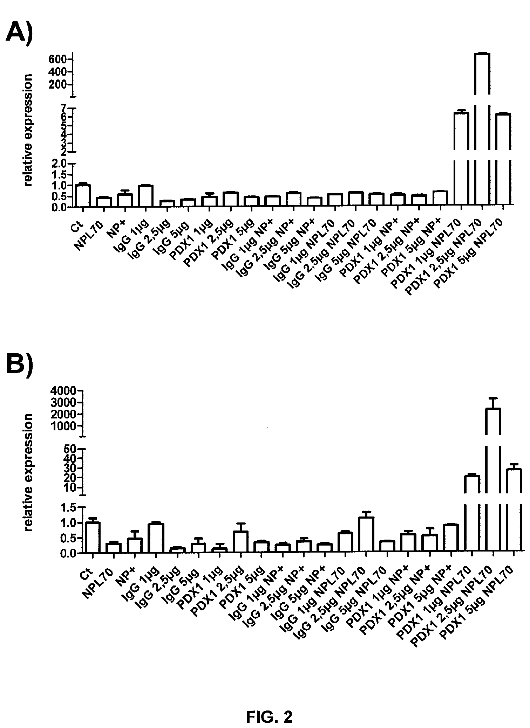

Comme représenté sur la figure 1, il n'y a aucun effet sur les gènes Ins1 et Ins2, 48h après l'introduction de l'anticorps à l'aide des nanoparticules NP+ ou des nanoparticules NPL70 dans la cellule. Toutefois, si on prolonge cette incubation jusqu'à 72h, on observe alors une forte augmentation de l'expression des gènes Ins1 et Ins2, et ceci uniquement quand on utilise les nanoparticules NPL70. Cet effet est spécifique pour l'expression de ces deux gènes, expression qui ne varie pas significativement après incubation de cellules avec les nanoparticules ou l'anticorps anti-PDX1 seuls (Figure 2). En conséquences, ces résultats démontrent que l'anticorps anti-PDX1 a été délivré dans la cellule et a interagi avec les gènes Ins1 et Ins2.

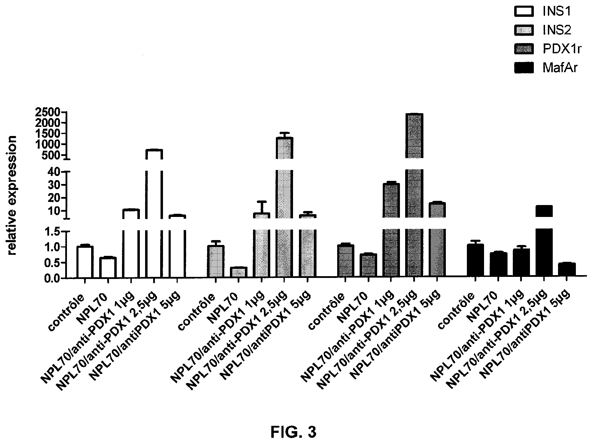

Il est connu que le facteur de transcription Pdx1, en plus de réguler les gènes Ins1 et Ins2, régule également l'expression de son gène par une boucle d'autorégulation ainsi que l'expression d'un autre facteur de transcription spécifique des cellules beta, le facteur Mafa. Il a donc été recherché l'effet de l'inhibition de l'activité de Pdx1 sur l'expression de ces gènes. La figure 3 démontre que l'expression des gènes Pdx1 et Mafa (à 72h) suit, comme attendu, un profil d'expression similaire aux gènes Ins1 et Ins2, ce qui signifie que l'anticorps a été effectivement délivré dans la cellule et agit sur le métabolisme de cette dernière.

Les exemples précités montrent d'une manière inattendue que la nanoparticule NPL70 permet la délivrance intracellulaire de l'anticorps anti-PDX1, alors que ce même anticorps soit seul, soit associé à la particule NP+ n'est pas délivré dans la cellule (aucun effet sur les gènes Ins1, Ins2, Pdx1 et Mafa testés).

De plus, contrairement à ce qui est généralement observé pour les autres techniques de transfection, la délivrance de l'anticorps anti-PDX1 dans les cellules n'affecte pas leur viabilité (aucune augmentation de la mortalité cellulaire n'a été observée).

### Détermination de la position de l'anticorps par rapport au coeur de la nanoparticule

La variation de la taille de la nanoparticule et la variation du potentiel zêta (charge électrique) des nanoparticules poreuses contenant un phospholipide (NPL70) a été étudiée en fonction de l'association de la nanoparticule avec de l'albumine de sérum bovin (BSA), de l'ovalbumine (OVA) et de l'anticorps anti-transferrine.

Lorsque la protéine pénètre dans le coeur de la nanoparticule, on observe une certaine stabilité de la taille de la nanoparticule (le coeur a tendance à gonfler tout de même) et une variation faible du potentiel zêta.

Lorsque la protéine vient se fixer sur le coeur de la nanoparticule, on observe une augmentation de taille de la nanoparticule et une diminution nette du potentiel zêta.

Une solution de NPL à 5mg/mL, une solution de BSA ou d'OVA à 1mg/ml et une solution contenant l'anticorps anti-transferrine à 0,5mg/mL (IgG1, Invitrogen) ont été utilisées.

L'association nanoparticule/protéine se fait par un simple mélange entre les NPL70 et les protéines. L'association est calculée en µg de protéine/µg de NPL70 (poids/poids, abrégé w/w dans les figures).

L'analyse de la taille et du potentiel zêta est réalisée à l'aide du ZetaSizer Nano (Malvern ® Instruments). La taille est mesurée dans une solution de NaCl 15mM. Le potentiel zêta est analysé dans l'eau.

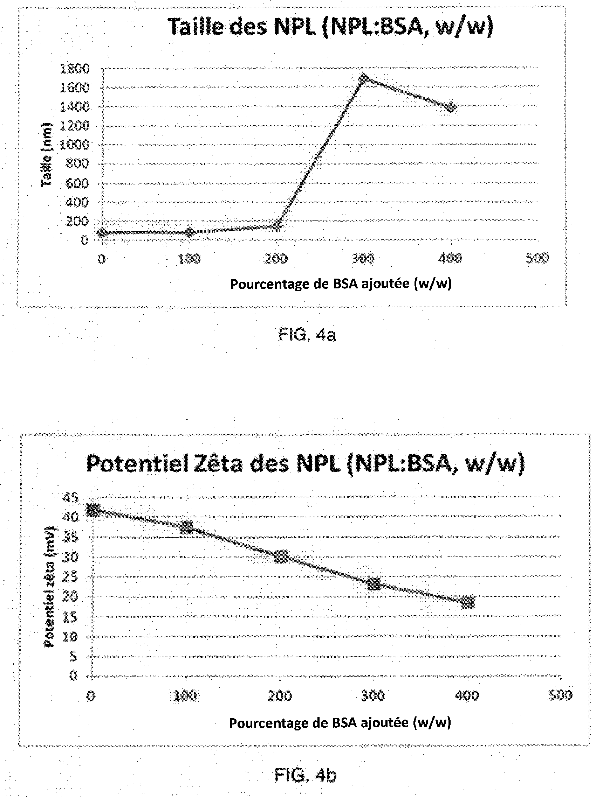

La Fig. 4a montre que la taille ne varie pas de façon significative lors de l'ajout de BSA. Une augmentation de la taille se produit lorsqu'on atteint un rapport NPL70/BSA de 200% ce qui suggère une saturation du coeur de la particule en protéine.

De même, comme visible sur la Fig. 4b, on observe que le potentiel zêta diminue progressivement ; sa diminution est visible après ajout de 200 µg de BSA, ce qui suggère que la protéine pénètre dans le coeur de la nanoparticule engendrant ainsi une faible modification de la charge de surface de cette dernière.

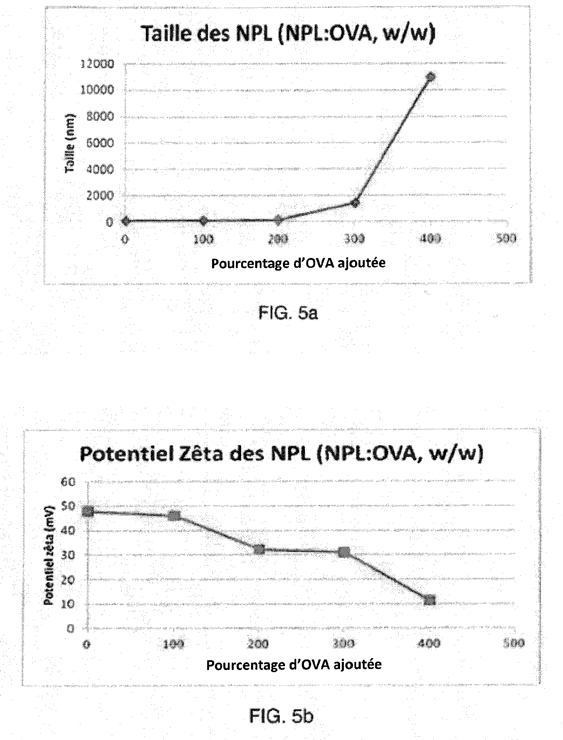

La Fig. 5a montre que la taille ne varie pas de façon significative lors de l'ajout d'OVA. Une variation se produit lorsqu'on atteint un rapport NPL/OVA de 300% ce qui suggère une saturation du coeur de la particule en protéine. De même, comme visible sur la Fig. 5b, on observe que le potentiel zêta diminue faiblement et de manière progressive ; sa diminution est visible après ajout de 200 % d'OVA. Ces résultats montrent que dans le cas de la BSA et de l'OVA, la protéine pénètre d'abord dans le coeur de la nanoparticule, le sature puis arrive en surface modifiant ainsi la charge de la nanoparticule.

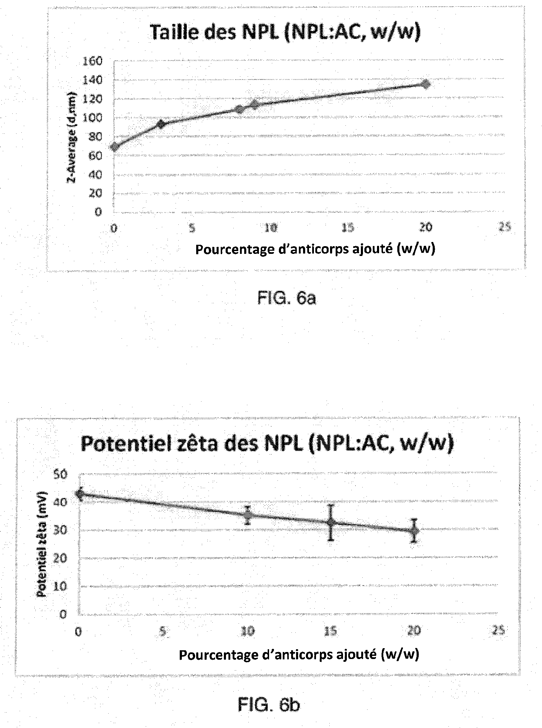

La Fig. 6a montre les résultats obtenus après association des NPL70 avec l'anticorps anti-transferrine (AC). Contrairement aux autres protéines de taille moins importante on observe qu'avec des % très faibles d'AC, la taille de la nanoparticule augmente. Cette augmentation est régulière en fonction de la quantité d'anticorps ajoutée. On en déduit que l'anticorps se lie à la surface du coeur chargé en lipide mais ne pénètre pas ou très peu dans le coeur. Ce résultat est corroboré par l'analyse du potentiel zéta. Comme représenté sur la Fig. 6b, le potentiel zêta diminue de manière significative et ceci avec un rapport NPL/AC de 10% d'AC alors qu'un résultat similaire est observé avec l'OVA ou la BSA avec 10 fois plus de protéine. Ce résultat suggère l'association en surface de l'AC qui étant négatif diminue la charge de surface des NPL70.

Les résultats précités montrent que les NPL70 dont le coeur est poreux et chargé en phospholipide sont capables d'emmagasiner dans leur coeur une grande quantité de protéine (en plus du phospholipide) comme la BSA ou l'OVA qui sont des protéines de petite taille (MwBSA : 69293 Da/ MwOVA : 42881 Da). En revanche, la porosité du coeur des nanoparticules limite l'accès de protéines plus importantes, comme par exemple, les anticorps (Mw Ac : 150000 Da). Ainsi, on observe que l'association des nanoparticules précitées avec des anticorps aboutit à une variation du diamètre moyen de la nanoparticule d'environ 12 nm. Alors que l'association avec l'OVA et la BSA qui entrent dans le coeur et gonfle ce dernier conduit à une augmentation de taille de 7 nm.

## Revendications

1. Association comprenant une nanoparticule et une protéine ou un fragment de protéine, ladite nanoparticule comprenant (a) un coeur solide et poreux, formé d'un polymère obtenu par réaction entre une maltodextrine et un composé époxyde, des fonctions ammonium quaternaire étant greffées sur ledit polymère pour lui conférer sa charge positive et (b) un lipide de charge ionique négative contenu dans ledit coeur, ladite association étant **caractérisée en ce que** ladite protéine ou ledit fragment de protéine est fixé(e) sur ledit coeur et **en ce que** ladite protéine ou ledit fragment de protéine est un anticorps ou un fragment fonctionnel d'anticorps.

2. Association selon la revendication 1, **caractérisée en ce que** ladite protéine est un anticorps.

3. Association selon la revendication 2, **caractérisée en ce que** ledit anticorps est un anticorps de type IgG, plus particulièrement l'anticorps PDX-1.

4. Association selon l'une quelconque des revendications 1 à 3, **caractérisée en ce que** ledit phospholipide est choisi parmi : le 1, 2-diacylyl-sn-glycéro-3-phosphatidylglycérol, le 1, 2-diacylyl-sn-glycero-3-phosphatidylsérine, le 1, 2-dipalmitoyl-sn-glycéro-3-phosphatidylglycérol et le 1, 2-diacylyl-sn-glycéro-3-phosphatidylsérine.

5. Utilisation *in vitro* d'une association entre une nanoparticule et un anticorps ou un fragment fonctionnel d'anticorps, selon l'une quelconque des revendications 1 à 4 pour la délivrance fonctionnelle de ladite protéine ou dudit fragment de protéine dans le cytosol d'une cellule, ladite protéine ou ledit fragment de protéine ainsi libéré dans le cytosol interagissant avec le fonctionnement de ladite cellule.

6. Procédé de délivrance *in vitro* d'un anticorps ou d'un fragment fonctionnel d'anticorps dans le cytosol d'une cellule, **caractérisé en ce que**
- on mélange, éventuellement dans un solvant adapté, un anticorps ou un fragment d'anticorps avec une nanoparticule qui comprend (a) un coeur solide et poreux, formé d'un polymère obtenu par réaction entre une maltodextrine et un composé époxyde, des fonctions ammonium quaternaire étant greffées sur ledit polymère pour lui conférer sa charge positive et (b) un lipide de charge ionique négative contenu dans ledit coeur ; et
- on met en contact ledit mélange avec une cellule vivante.

7. Procédé de délivrance selon la revendication 6, **caractérisé en ce que** ledit phospholipide est choisi parmi le 1, 2-diacylyl-sn-glycéro-3-phosphatidylglycérol, le 1, 2-diacylyl-sn-glycero-3-phosphatidylsérine, le 1, 2-dipalmitoyl-sn-glycéro-3-phosphatidylglycérol et le 1, 2-diacylyl-sn-glycéro-3-phosphatidylsérine.

8. Procédé selon la revendication 6 ou 7, **caractérisé en ce que** ledit anticorps ou ledit fragment fonctionnel est un anticorps.

9. Procédé selon l'une quelconque des revendications 6 à 8, **caractérisé en ce que** ledit anticorps est un anticorps de type IgG, plus particulièrement, l'anticorps PDX-1.

10. Procédé selon l'une quelconque des revendications 6 à 9, **caractérisé en ce que** ledit solvant est choisi parmi les solvants protiques polaires, en particulier l'eau et les solvants protiques apolaires.

11. Composition pharmaceutique comprenant, en tant que composant actif, une association de nanoparticule avec un anticorps ou un fragment fonctionnel d'anticorps selon l'une quelconque des revendications 1 à 4 et un solvant pharmaceutiquement acceptable.

## Patentansprüche

1. Kombination bestehend aus einem Nanoteilchen und einem Protein oder Proteinfragment, wobei besagtes Nanoteilchen (a) einen festen und durchlässigen Kern umfasst, der gebildet wird aus einem Polymer, das durch eine Reaktion zwischen einem Maltodextrin und einer Epxoydverbindung erzielt wird, wobei Quaternärammoniumgruppen auf besagtes Polymer gepfropft wurden, um ihm seine positive Ladung zu verleihen, (b) ein Lipid mit negativer ionischer Ladung umfasst, das in besagtem Kern enthalten ist, wobei besagte Kombination **dadurch gekennzeichnet ist, dass** besagtes Protein oder besagtes Proteinfragment auf besagtem Kern fixiert ist und dadurch, dass besagtes Protein oder besagtes Proteinfragment ein Antikörper oder ein funktionales Antikörperfragment ist.

2. Kombination nach Anspruch 1, **dadurch gekennzeichnet, dass** besagtes Protein ein Antikörper ist.

3. Kombination nach Anspruch 2, **dadurch gekennzeichnet, dass** besagter Antikörper ein Antikörper Typ IgB, und insbesondere der Antikörper PDX-1 ist.

4. Kombination nach einem der Ansprüche 1 bis 3, **dadurch gekennzeichnet, dass** besagtes Phospholipid ausgewählt wird aus: 1, 2 Diacylyl-sn-Glycero-3-Phosphatidylglycerol, 1, 2-Diacylyl-sn-Glycero-3-Phosphatidylserin, 1, 2-Dipalmitoyl-sn-Glycero-3-Phosphatidylglycerol und 1, 2-Diacylyl-sn-Glycero-3-Phosphatidylserin.

5. In Vitro-Benutzung einer Kombination bestehend aus einem Nanoteilchen und einem Antikörper oder einem funktionalen Antikörperfragment nach einem der Ansprüche 1 bis 4 für die funktionale Abgabe besagten Proteins oder besagten Proteinfragments in das Cytosol einer Zelle, wobei das auf diese Weise im Cytosol freigesetzte Protein oder Proteinfragment mit der Funktionsweise der besagten Zelle in Wechselwirkung steht.

6. Verfahren zur In Vitro-Abgabe eines Antikörpers oder eines funktionalen Antikörperfragments in das Cytosol einer Zelle, **dadurch gekennzeichnet, dass** man
- eventuell in einem geeigneten Lösungsmittel einen Antikörper oder ein Antikörperfragment mit einem Nanoteilchen mischt, das (a) einen festen und durchlässigen Kern umfasst, der gebildet wird aus einem Polymer, das durch eine Reaktion zwischen einem Maltodextrin und einer Epxoydverbindung erzielt wird, wobei Quaternärammoniumgruppen auf besagtes Polymer gepfropft werden, um ihm seine positive Ladung zu verleihen, (b) ein Lipid mit negativer ionischer Ladung umfasst, das in besagtem Kern enthalten ist, und
- man besagte Mischung mit einer lebenden Zelle in Kontakt bringt.

7. Abgabeverfahren nach Anspruch 6, **dadurch gekennzeichnet, dass** besagtes Phospholipid ausgewählt wird aus 1, 2 Diacylyl-sn-Glycero-3-Phosphatidylglycerol, 1, 2-Diacylyl-sn-Glycero-3-Phosphatidylserin, 1, 2-Dipalmitoyl-sn-Glycero-3-Phosphatidylglycerol und 1, 2-Diacylyl-sn-Glycero-3-Phosphatidylserin.

8. Verfahren nach Anspruch 6 oder 7, **dadurch gekennzeichnet, dass** besagter Antikörper oder besagtes funktionales Fragment ein Antikörper ist.

9. Verfahren nach einem der Ansprüche 6 bis 8, **dadurch gekennzeichnet, dass** besagter Antikörper ein Antikörper vom Typ IgG ist und insbesondere ein Antikörpers PDX-1.

10. Verfahren nach einem der Ansprüche 6 bis 9, **dadurch gekennzeichnet, dass** besagtes Lösungsmittel ausgewählt wird aus polaren protischen Lösungsmitteln, insbesondere Wasser, und apolaren protischen Lösungsmitteln.

11. Pharmazeutische Zusammensetzung, welche als Wirkstoff eine Kombination aus einem Nanoteilchen und einem Antikörper oder einem funktionalen Antikörperfragment nach einem der Ansprüche 1 bis 4 und ein pharmazeutisch akzeptables Lösungsmittel enthält.

## Claims

1. A combination comprising a nanoparticle and a protein or a protein fragment, with said nanoparticle comprising (a) a porous solid core, formed of a polymer obtained by reaction between a maltodextrin and an epoxy compound, with quaternary ammonium functions being grafted onto said polymer to impart its positive charge and (b) a lipid having a negative ionic charge contained in said core, with said combination being **characterized in that** said protein or said protein fragment is attached on said core, and **in that** said protein or said protein fragment is an antibody or a functional fragment of an antibody.

2. A combination according to claim 1, **characterized in that** said protein is an antibody.

3. A combination according to claim 2, **characterized in that** said antibody is an antibody of the IgG type, more particularly the PDX-1 antibody.

4. A combination according to any one of claims 1 to 3, **characterized in that** said phospholipid is selected from: 1,2-diacylyl-sn-glycero-3-phosphatidylglycerol, 1,2-diacylyl-sn-glycero-3-phosphatidylserine, 1,2-dipalmitoyl-sn-glycero-3-phosphatidylglycerol and 1,2-diacylyl-sn-glycero-3-phosphatidylserine.

5. *In vitro* use of a combination between a nanoparticle and an antibody or a functional fragment of an antibody according to any one of claims 1 to 4 for the functional delivery of said protein or said protein fragment into the cytosol of a cell, with said protein or said protein fragment thus delivered into the cytosol interacting with the operation of said cell.

6. A method for the *in vitro* delivery of an antibody or a functional fragment of an antibody into the cytosol of a cell, **characterized in that**
- an antibody or a functional fragment of an antibody is mixed, optionally in a suitable solvent, with a nanoparticle which comprises (a) a porous solid core, formed of a polymer obtained by reaction between a maltodextrin and an epoxy compound, with quaternary ammonium functions being grafted onto said polymer to impart its positive charge and (b) a lipid having a negative ionic charge contained in said core; and
- said mixture is brought into contact with a living cell.

7. A delivery method according to claim 6, **characterized in that** said phospholipid is selected from 1,2-diacylyl-sn-glycero-3-phosphatidylglycerol, 1,2-diacylyl-sn-glycero-3-phosphatidylserine, 1,2-dipalmitoyl-sn-glycero-3-phosphatidylglycerol and 1,2-diacylyl-sn-glycero-3-phosphatidylserine.

8. A method according to claim 6 or 7, **characterized in that** said antibody or said functional fragment is an antibody.

9. A method according to any one of claims 6 to 8, **characterized in that** said antibody is an antibody of the IgG type and, more particularly, the PDX-1 antibody.

10. A method according to any one of claims 6 to 9, **characterized in that** said solvent is selected from polar protic solvents, in particular water and protic apolar solvents.

11. A pharmaceutical composition comprising, as the active component, a combination of a nanoparticle with an antibody or a functional fragment of an antibody according to any one of claims 1 to 4 and a pharmaceutically acceptable solvent.
